# EUROPEAN PATENT APPLICATION

(11) **EP 4 792 862 A1**
(43) Date of publication of application: **19.08.2026**
(21) Application number: 24876215.5
(22) Date of filing: 08.08.2024
(51) Int. Cl.: A61M 16/00

(54) **PARAMETER ADJUSTMENT METHOD AND APPARATUS, VENTILATION THERAPY DEVICE, AND STORAGE MEDIUM**

(30) Priority: 13.10.2023 CN 202311330669
(71) Applicant: BMC Medical Co., Ltd., Beijing 100089 (CN)
(72) Inventor: ZHANG, Lixin, Beijing 100089 (CN); ZHANG, Anjun, Beijing 100089 (CN); LAN, Guanglei, Beijing 100089 (CN); WANG, Qingsong, Beijing 100089 (CN); ZHUANG, Zhi, Beijing 100089 (CN)
(74) Representative: Grünecker Patent- und Rechtsanwälte PartG mbB
(86) International application number: PCT/CN2024/110682
(87) International publication number: WO 2025/077395

(57) **Abstract**

The present application relates to a parameter adjustment method and apparatus, a ventilation therapy device, and a storage medium, and pertains to the technical field of ventilation therapy devices. The parameter adjustment method is applied to a ventilation therapy device. The method includes: receiving a first trigger operation by a user on a target scheme control, where a first display interface of the ventilation therapy device includes at least one scheme control, and the target scheme control is one of the at least one scheme control; and performing, in response to the first trigger operation, parameter adjustment for the ventilation therapy device according to a target parameter scheme corresponding to the target scheme control, where the target parameter scheme includes a target parameter and a parameter setting value corresponding to the target parameter. The parameter adjustment method allows a user to conveniently perform parameter adjustment for the ventilation therapy device according to the target parameter scheme by triggering the target scheme control. The operation is simple and convenient, and the efficiency in parameter adjustment for the ventilation therapy device can be improved.

## Description

### CROSS-REFERENCE TO RELATED APPLICATIONS

This application claims priority to Chinese Patent Application No. 202311330669.2, filed with the China National Intellectual Property Administration on October 13, 2023 and entitled "PARAMETER ADJUSTMENT METHOD AND APPARATUS, VENTILATION THERAPY DEVICE, AND STORAGE MEDIUM", the entire contents of which are incorporated herein by reference.

### TECHNICAL FIELD

The present application relates to the technical field of ventilation therapy devices, and in particular to a parameter adjustment method and apparatus, a ventilation therapy device, and a storage medium.

### BACKGROUND

For a ventilation therapy device such as a high-flow humidified oxygen therapy device or a ventilator, parameter values of parameters such as flow rate, oxygen concentration, and temperature of the ventilation therapy device can be adjusted so that the ventilation therapy device operates under different device parameters.

In the related art, when performing parameter adjustment for a ventilation therapy device, a user is required to manually adjust and set numerical values of device parameters according to actual needs, which is cumbersome and leads to low efficiency in parameter adjustment for existing ventilation therapy devices.

### SUMMARY

The present application provides a parameter adjustment method and apparatus, a ventilation therapy device, and a storage medium, to solve the problem of low efficiency in parameter adjustment of existing ventilation therapy devices.

The technical solutions of the present application are as follows:
According to a first aspect of some embodiments of the present application, a parameter adjustment method applied to a ventilation therapy device is provided. The method includes:
receiving a first trigger operation by a user on a target scheme control, where a first display interface of the ventilation therapy device includes at least one scheme control, and the target scheme control is one of the at least one scheme control; and
performing, in response to the first trigger operation, parameter adjustment for the ventilation therapy device according to a target parameter scheme corresponding to the target scheme control, where the target parameter scheme includes a target parameter and a parameter setting value corresponding to the target parameter.

In some embodiments, after performing, in response to the first trigger operation, the parameter adjustment for the ventilation therapy device according to the target parameter scheme corresponding to the target scheme control, the method further includes:
displaying a second display interface in response to the first trigger operation, where the second display interface includes a target parameter control corresponding to the target parameter, and the target parameter control is configured to display a parameter setting value and a latest monitoring value of the corresponding target parameter.

In some embodiments, the ventilation therapy device includes at least one parameter mode; the ventilation therapy device, in any one of the parameter modes, automatically operates according to a corresponding preset parameter scheme; and performing, in response to the first trigger operation, the parameter adjustment for the ventilation therapy device according to the target parameter scheme corresponding to the target scheme control includes:
adjusting the ventilation therapy device to operate in a target parameter mode in response to the first trigger operation, where the target parameter mode is a parameter mode, among the at least one parameter mode, that is associated with the target scheme control.

In some embodiments, the first display interface further includes a create control, and the method further includes:
receiving a second trigger operation by the user on the create control;
displaying a scheme selection interface in response to the second trigger operation, where the scheme selection interface includes a preset scheme control, and the preset scheme control includes a parameter settings list;
receiving a third trigger operation by the user on the preset scheme control; and
performing, in response to the third trigger operation, parameter adjustment for the ventilation therapy device according to the parameter settings list of the preset scheme control.

In some embodiments, the first display interface further includes an alarm control, and the method further includes:
receiving a fourth trigger operation by the user on the alarm control;
displaying an alarm settings interface in response to the fourth trigger operation, where the alarm settings interface includes an alarm item control;
receiving a fifth trigger operation by the user on the alarm item control; and
updating a parameter alarm threshold corresponding to the alarm item control in response to the fifth trigger operation.

In some embodiments, the first display interface further includes a log control, and the method further includes:
receiving a sixth trigger operation by the user on the log control;
displaying a log interface in response to the sixth trigger operation, where the log interface includes a log item control;
receiving a seventh trigger operation by the user on the log item control; and
displaying log item information corresponding to the log item control in response to the seventh trigger operation.

In some embodiments, the first display interface further includes a trend chart control, and the method further includes:
receiving an eighth trigger operation by the user on the trend chart control;
displaying a trend chart interface in response to the eighth trigger operation, where the trend chart interface includes: a time interval control and a parameter graph control corresponding to the target parameter;
receiving a ninth trigger operation by the user on the time interval control; and
displaying, in the parameter graph control in response to the ninth trigger operation, a parameter value variation trend of the corresponding target parameter over a target time interval, where the target time interval is a time interval currently displayed by the time interval control.

According to a second aspect of some embodiments of the present application, a parameter adjustment apparatus applied to a ventilation therapy device is provided. The apparatus includes:
a first receiving module configured to receive a first trigger operation by a user on a target scheme control, where a first display interface of the ventilation therapy device includes at least one scheme control, and the target scheme control is one of the at least one scheme control; and
a first adjustment module configured to perform, in response to the first trigger operation, parameter adjustment for the ventilation therapy device according to a target parameter scheme corresponding to the target scheme control, where the target parameter scheme includes a target parameter and a parameter setting value corresponding to the target parameter.

According to a third aspect of some embodiments of the present application, a ventilation therapy device is provided. The ventilation therapy device is equipped with the parameter adjustment apparatus according to the second aspect, and is configured to perform the parameter adjustment method according to the first aspect.

According to a fourth aspect of some embodiments of the present application, a storage medium is provided. Instructions in the storage medium, when executed by a processor of an electronic device, cause the electronic device to perform the parameter adjustment method according to any one of the embodiments of the first aspect.

The technical solutions provided in some embodiments of the present application bring at least the following beneficial effects:
In some embodiments of the present application, the first trigger operation by the user on the target scheme control is received. The first display interface corresponding to the ventilation therapy device includes the at least one scheme control, and the target scheme control is one of the at least one scheme control. Therefore, the user can conveniently select, according to needs, one of the parameter schemes corresponding to the scheme controls as the target parameter scheme corresponding to the target scheme control, and perform parameter adjustment for the ventilation therapy device according to the target parameter scheme. The target parameter scheme includes the target parameter and the parameter setting value corresponding to the target parameter. In this way, a latest parameter value of the target parameter of the ventilation therapy device after the adjustment can match the parameter setting value in the target parameter scheme, thereby accomplishing a parameter adjustment operation of the ventilation therapy device by the user. In the case where the target parameter scheme includes a plurality of target parameters, parameter setting values of the plurality of target parameters of the ventilation therapy device can also be adjusted with one click in response to the first trigger operation by the user on the target scheme control. Therefore, the problem of cumbersome operation can be avoided. Moreover, compared with an operation in the related art where the user is required to manually adjust and set parameter values of device parameters, the parameter adjustment method provided in some embodiments of the present application only requires the user to trigger the target scheme control to perform parameter adjustment for the ventilation therapy device according to the target parameter scheme. The operation is simple and convenient. Therefore, the efficiency in parameter adjustment for the ventilation therapy device can be improved.

It should be understood that the above general description and the following detailed description are merely exemplary and explanatory, and do not limit the present application.

### BRIEF DESCRIPTION OF THE DRAWINGS

The accompanying drawings herein, which are incorporated into the description and constitute a part of the description, illustrate some embodiments in accordance with the present application and are used together with the description to explain the principles of the present application, and do not unduly limit the present application.
FIG. 1 is a first flowchart showing a parameter adjustment method according to some embodiments;
FIG. 2 is a first schematic diagram showing a first display interface according to some embodiments;
FIG. 3 is a second flowchart showing a parameter adjustment method according to some embodiments;
FIG. 4 is a schematic diagram showing a second display interface according to some embodiments;
FIG. 5 is a schematic diagram showing a scheme selection interface according to some embodiments;
FIG. 6 is a schematic diagram showing an alarm settings interface according to some embodiments;
FIG. 7 is a schematic diagram showing a log interface according to some embodiments;
FIG. 8 is a schematic diagram showing a trend chart interface according to some embodiments;
FIG. 9 is a schematic diagram showing a settings interface according to some embodiments;
FIG. 10 is a schematic diagram showing a menu page according to some embodiments;
FIG. 11 is a second schematic diagram showing a first display interface according to some embodiments; and
FIG. 12 is a block diagram showing a parameter adjustment apparatus according to some embodiments.

### DETAILED DESCRIPTION

In order for a person of ordinary skill in the art to better understand the technical solutions of the present application, the technical solutions in some embodiments of the present application will be clearly and completely described below with reference to the accompanying drawings.

It should be noted that the terms "first", "second" and the like in the description and claims of the present application as well as the above-mentioned accompanying drawings are used to distinguish similar objects, and are not necessarily used to describe a specific sequence or order of precedence. It should be understood that the data termed in this way may be interchanged where appropriate, so that some embodiments of the present application described herein can be implemented in a sequence other than those illustrated or described herein. The implementations described below in some exemplary embodiments do not represent all implementations consistent with the embodiments of the present application. Instead, the implementations are merely examples of apparatuses and methods consistent with some aspects of the present application, as detailed in the appended claims.

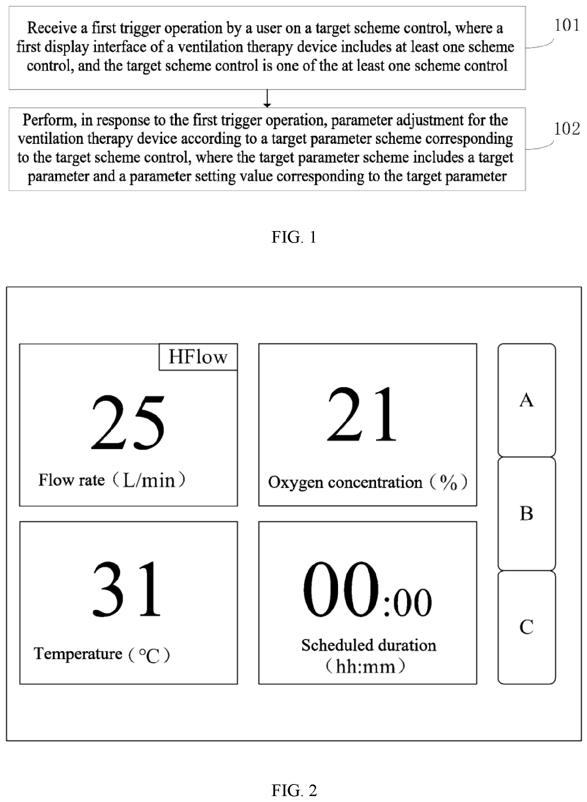

FIG. 1 is a first flowchart showing a parameter adjustment method according to some embodiments. As shown in FIG. 1, the parameter adjustment method is applied to a ventilation therapy device, and may include the following steps.

Step 101: Receive a first trigger operation by a user on a target scheme control, where a first display interface of the ventilation therapy device includes at least one scheme control, and the target scheme control is one of the at least one scheme control.

In some embodiments of the present application, the ventilation therapy device may be a high-flow humidified oxygen therapy device, a ventilator, or an integrated machine having both a high-flow mode and a ventilator mode, as well as a medical oxygen concentrator, etc. This is by way of example only, and some embodiments of the present application are not limited thereto.

In some embodiments of the present application, the ventilation therapy device may include a display screen, such as a liquid crystal display (LCD) screen. The first display interface may be displayed on the display screen of the ventilation therapy device. In the first display interface, the number of scheme controls may be one or more, and is not limited in some embodiments of the present application. For any one of the scheme controls, the scheme control may include a corresponding scheme label, and the corresponding scheme label is displayed in the scheme control. For example, the first display interface includes three scheme controls, with scheme labels "A", "B", and "C" displayed, respectively. This is by way of example only, and some embodiments of the present application are not limited thereto.

In some embodiments of the present application, the first trigger operation may be an operation of the user clicking the target scheme control. Specifically, the user may perform the trigger operation on the target scheme control using an input device connected to the ventilation therapy device. For example, the input device connected to the ventilation therapy device may be a mouse or a keyboard, and the user may manipulate the mouse to click the target scheme control, or may trigger the target scheme control by entering a shortcut via the keyboard. This is by way of example only, and some embodiments of the present application are not limited thereto.

In some embodiments of the present application, the display screen of the ventilation therapy device may be a touch-sensitive display screen, and the first trigger operation may be an operation of the user tapping the target scheme control on the first display interface of the display screen. Alternatively, a specific form of the first trigger operation may be set according to actual conditions of the ventilation therapy device and needs of the user, and is not limited in some embodiments of the present application.

Step 102: Perform, in response to the first trigger operation, parameter adjustment for the ventilation therapy device according to a target parameter scheme corresponding to the target scheme control, where the target parameter scheme includes a target parameter and a parameter setting value corresponding to the target parameter.

In some embodiments of the present application, the target parameter may be a device parameter of the ventilation therapy device. The ventilation therapy device may include one or more target parameters, and a plurality of parameter schemes can be obtained by setting the target parameters to different parameter values. The number of parameter schemes of the ventilation therapy device is not less than the number of scheme controls on the first display interface. In the case where the first display interface includes only one scheme control, the scheme control may be the target scheme control. In the case where the first display interface includes a plurality of scheme controls, the plurality of scheme controls may be respectively associated with different parameter schemes, so that the scheme controls respectively correspond to different parameter schemes. For example, the scheme control A, the scheme control B, and the scheme control C may respectively correspond to a parameter scheme A, a parameter scheme B, and a parameter scheme C of the ventilation therapy device.

For example, for a ventilation therapy device such as a high-flow humidified oxygen therapy device or a ventilator, the target parameters may include flow rate, oxygen concentration, and temperature of output gas. The number of target parameters may be one or more. This is by way of example only, and some embodiments of the present application are not limited thereto.

In some embodiments of the present application, in response to the first trigger operation by the user, a parameter scheme associated with the target scheme control may be determined as the target parameter scheme. Then, parameter adjustment is performed for the ventilation therapy device according to the target parameter scheme. Specifically, for any target parameter in the target parameter scheme, a current actual value of the target parameter of the ventilation therapy device may be adjusted according to a parameter setting value corresponding to the target parameter, to obtain an adjustment result of the target parameter. The adjustment result may be a latest monitoring value of the target parameter.

In some embodiments of the present application, the parameter scheme of the ventilation therapy device may be a custom parameter scheme generated by the user presetting parameter values for the target parameters. Alternatively, the ventilation therapy device may have an intrinsic parameter mode. When the ventilation therapy device is switched to operate in any parameter mode, the ventilation therapy device can automatically adjust parameter values of the target parameters according to a parameter scheme preset for the parameter mode, i.e., a preset parameter scheme, and start device operation.

For example, in an anesthesia application scenario, the ventilation therapy device may be a high-flow humidified oxygen therapy device. For different phases of anesthesia, such as a preoxygenation phase, an apneic oxygenation period, and a regular therapy phase, the ventilation therapy device is required to operate under different device parameters. In the related art, a user is usually required to manually adjust and set device parameters to switch operation between the phases.

In some embodiments of the present application, the first display interface of the ventilation therapy device may include three scheme controls: a scheme control A, a scheme control B, and a scheme control C, corresponding to three phases of anesthesia: A - preoxygenation phase, B - apneic oxygenation period, and C - regular therapy phase, respectively. The parameter scheme A corresponding to the scheme control A may include: a flow rate of 30 liters per minute (L/min), an oxygen concentration of 100%, and a temperature of 34 degrees Celsius (°C). The parameter scheme B corresponding to the scheme control B may include: a flow rate of 70 L/min, an oxygen concentration of 100%, and a temperature of 37°C. The parameter scheme C corresponding to the scheme control C may include: a flow rate of 40 L/min, an oxygen concentration of 50%, and a temperature of 34°C. This is by way of example only, and the user may set specific numerical values of the device parameters according to actual needs. Some embodiments of the present application are not limited thereto.

In actual applications, the user may click or tap the scheme control A, the scheme control B, or the scheme control C correspondingly according to specific situations. In response to the first trigger operation by the user on the target scheme control, the ventilation therapy device may perform parameter scheme adjustment for the ventilation therapy device according to the parameter scheme A, the parameter scheme B, or the parameter scheme C, and start device operation. It should be noted that the preoxygenation phase is an important step before induction of general anesthesia, and its purpose is to completely remove nitrogen from the lungs, thereby increasing the body's oxygen reserves and prolonging the tolerable apnea time. The parameter adjustment method according to some embodiments of the present application allows the user to conveniently adjust the device parameters with one click through the trigger operation on the scheme control A, thereby avoiding possible errors caused by manual adjustment and setting by the user, improving the accuracy and efficiency of parameter adjustment, and enhancing the effect of use of the ventilation therapy device in the preoxygenation phase. It should be noted that, after the induction of general anesthesia, for a case of hypoxemia, the user can quickly adjust the device parameters of the ventilation therapy device to the parameter scheme B corresponding to the apneic oxygenation period through a trigger operation on the scheme control B, and start device operation, thereby reducing accident risk and improving usage safety of the ventilation therapy device.

In some embodiments, the ventilation therapy device includes at least one parameter mode; the ventilation therapy device, in any one of the parameter modes, automatically operates according to a corresponding preset parameter scheme; and performing, in response to the first trigger operation, the parameter adjustment for the ventilation therapy device according to the target parameter scheme corresponding to the target scheme control includes:
adjusting the ventilation therapy device to operate in a target parameter mode in response to the first trigger operation, where the target parameter mode is a parameter mode, among the at least one parameter mode, that is associated with the target scheme control.

In some embodiments of the present application, the parameter modes of the ventilation therapy device may include: a parameter mode that operates according to a fixed parameter value, a parameter mode that automatically adjusts a parameter setting value within a preset numerical value range, a parameter mode that automatically adjusts a parameter setting value according to a preset condition, and a parameter mode that automatically adjusts a parameter setting value according to monitoring data of the ventilation therapy device. For any one of the parameter modes, a parameter scheme may be preset according to characteristics of the parameter mode; that is, a preset parameter scheme corresponding to the parameter mode is obtained. The preset parameter scheme includes one or more target parameters. The target parameters included in the preset parameter scheme may be determined according to actual needs, and preset to default parameter values. When the ventilation therapy device is switched to any one of the parameter modes, parameter adjustment may be performed for the ventilation therapy device according to the preset parameter scheme corresponding to the parameter mode, and device operation may be started, thereby enabling the ventilation therapy device to automatically operate in the parameter mode according to the corresponding preset parameter scheme.

For example, for a high-flow humidified oxygen therapy device, the plurality of parameter modes may include: high flow (HFlow) mode, low flow (LFlow) mode, auto flow (AutoFlow) mode, smart flow (SmartFlow) mode, etc. For example, for a ventilator, the plurality of parameter modes may include: spontaneous/timed (S/T) mode, continuous positive airway pressure (CPAP) mode, pressure controlled ventilation (PCV) mode, etc. For example, for an integrated machine, the plurality of parameter modes may include: the parameter modes of the high-flow humidified oxygen therapy device, the parameter modes of the ventilator, a custom parameter mode of the user, etc. The parameter modes of the integrated machine may be combined by the user according to actual needs. This is by way of example only, and some embodiments of the present application are not limited thereto.

In some embodiments of the present application, for a scheme control, the scheme control may be associated with a parameter mode of the ventilation therapy device. In the case where the first display interface includes a plurality of scheme controls, the scheme controls may be respectively associated with different parameter modes. For example, the scheme control A, the scheme control B, and the scheme control C may respectively correspond to high flow (HFlow) mode, auto flow (AutoFlow) mode, and low flow (LFlow) mode of the high-flow humidified oxygen therapy device. Alternatively, for the ventilator, the scheme control A, the scheme control B, and the scheme control C may correspond to auto continuous positive airway pressure (AutoCPAP) mode, spontaneous/timed (S/T) mode, and assured pressure support (SVAPS) mode, respectively. This is by way of example only, and some embodiments of the present application are not limited thereto.

In some embodiments of the present application, in response to the first trigger operation by the user, the parameter mode associated with the target scheme control may be determined as the target parameter mode, and then the ventilation therapy device may be adjusted to operate in the target parameter mode. Specifically, in response to the first trigger operation by the user, parameter adjustment may be performed for the ventilation therapy device according to the preset parameter scheme corresponding to the target parameter mode, and device operation may be started, thereby enabling the ventilation therapy device to automatically operate in the target parameter mode according to the corresponding preset parameter scheme.

In some embodiments of the present application, because the target parameter mode is a parameter mode, among the at least one parameter mode, that is associated with the target scheme control, the target parameter mode corresponding to the target scheme control can be determined in response to the first trigger operation by the user on the target scheme control. Then, the ventilation therapy device is adjusted to operate in the target parameter mode, so that the parameter mode of the ventilation therapy device after the adjustment meets requirements of the user. The ventilation therapy device includes the at least one parameter mode, and the ventilation therapy device automatically operates in any one of the parameter modes according to a corresponding preset parameter scheme. In this way, an association can be made between the scheme control and the parameter mode of the ventilation therapy device. This allows the user to conveniently select a desired parameter mode through a trigger operation on the scheme control, so that the ventilation therapy device can automatically operate according to the corresponding preset parameter scheme. Thus, the response speed of the ventilation therapy device can be improved, thereby enhancing the practicality of the parameter adjustment method of the present application.

FIG. 2 is a first schematic diagram showing a first display interface according to some embodiments. As shown in FIG. 2, the first display interface includes: a scheme control A, a scheme control B, and a scheme control C, respectively corresponding to a parameter scheme A, a parameter scheme B, and a parameter scheme C of the ventilation therapy device. Device parameters of the ventilation therapy device include flow rate, oxygen concentration, temperature, and scheduled duration; that is, target parameters include flow rate, oxygen concentration, temperature, and scheduled duration. A unit of the flow rate is liters per minute (L/min), a unit of the oxygen concentration is percent (%), a unit of the temperature is degrees Celsius (°C), and a time format of the scheduled duration is hh:mm, where "hh" represents hours in 12-hour format, and "mm" represents minutes in 12-hour format. In the case where a scheme control on the first display interface is associated with a parameter mode of the ventilation therapy device, a mode label, such as HFlow in FIG. 2, of the parameter mode may be displayed in the first display interface.

In some embodiments of the present application, the first trigger operation by the user on the target scheme control is received. The first display interface corresponding to the ventilation therapy device includes the at least one scheme control, and the target scheme control is one of the at least one scheme control. Therefore, the user can conveniently select, according to needs, one of the parameter schemes corresponding to the scheme controls as the target parameter scheme corresponding to the target scheme control, and perform parameter adjustment for the ventilation therapy device according to the target parameter scheme. The target parameter scheme includes the target parameter and the parameter setting value corresponding to the target parameter. In this way, a latest parameter value of each target parameter of the ventilation therapy device after the adjustment can match the parameter setting value in the target parameter scheme, thereby accomplishing a parameter adjustment operation of the ventilation therapy device by the user. In the case where the target parameter scheme includes a plurality of target parameters, parameter setting values of the plurality of target parameters of the ventilation therapy device can also be adjusted with one click in response to the first trigger operation by the user on the target scheme control. Therefore, the problem of cumbersome operation can be avoided. Moreover, compared with an operation in the related art where the user is required to manually adjust and set parameter values of device parameters, the parameter adjustment method provided in some embodiments of the present application only requires the user to trigger the target scheme control to perform parameter adjustment for the ventilation therapy device according to the target parameter scheme. The operation is simple and convenient. Therefore, the efficiency in parameter adjustment for the ventilation therapy device can be improved.

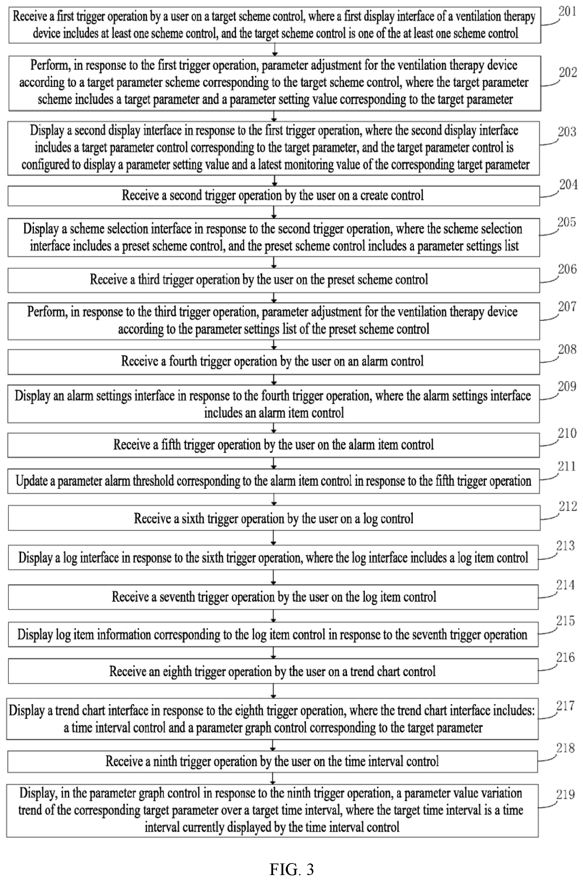

FIG. 3 is a second flowchart showing a parameter adjustment method according to some embodiments. As shown in FIG. 3, the parameter adjustment method is applied to a ventilation therapy device, and may include the following steps.

Step 201: Receive a first trigger operation by a user on a target scheme control, where a first display interface of the ventilation therapy device includes at least one scheme control, and the target scheme control is one of the at least one scheme control.

For a specific implementation of this step, reference may be made to the related description in the foregoing step 101, and details are not described herein again.

Step 202: Perform, in response to the first trigger operation, parameter adjustment for the ventilation therapy device according to a target parameter scheme corresponding to the target scheme control, where the target parameter scheme includes a target parameter and a parameter setting value corresponding to the target parameter.

For a specific implementation of this step, reference may be made to the related description in the foregoing step 102, and details are not described herein again.

Step 203: Display a second display interface in response to the first trigger operation, where the second display interface includes a target parameter control corresponding to the target parameter, and the target parameter control is configured to display a parameter setting value and a latest monitoring value of the corresponding target parameter.

In some embodiments of the present application, in response to the first trigger operation, parameter adjustment may be performed for the ventilation therapy device according to the target parameter scheme corresponding to the target scheme control, and the second display interface may be displayed on the display screen of the ventilation therapy device. The first display interface may be replaced with the second display interface to be displayed on the display screen of the ventilation therapy device. Alternatively, an interface size of the first display interface may be adjusted to make room for the second display interface, so that the display screen of the ventilation therapy device displays the first display interface and the second display interface simultaneously. The size and layout of the two display interfaces are not limited in some embodiments of the present application.

In some embodiments of the present application, the number of target parameter controls may be set according to needs of the user. Target parameter controls for all of the target parameters may be displayed in the first display interface, or target parameter controls for specific target parameters may be set. This is not limited in some embodiments of the present application. For any one of the target parameter controls, a parameter setting value to be displayed may be determined according to a parameter setting value of a target parameter corresponding to the target parameter control in the target parameter scheme, and a latest monitoring value of the target parameter of the ventilation therapy device may be obtained. The parameter setting value and the latest monitoring value of the target parameter are displayed in the target parameter control. The latest monitoring value of the target parameter may be obtained through measurement by a relevant sensor in the ventilation therapy device. Certainly, it may also be obtained by using other measurement methods. This is not limited in some embodiments of the present application.

It should be noted that step 202 and step 203 are both steps performed in response to the first trigger operation by the user on the target scheme control. Some embodiments of the present application do not limit an execution order of step 202 and step 203, and the user may determine the execution order according to actual needs.

In a feasible implementation, the second display interface may include a parameter display area and a data display area. The parameter display area includes the target parameter control, and the data display area includes a monitoring data control. The monitoring data control is configured to display monitoring data obtained by the ventilation therapy device, where the monitoring data may include data obtained by the ventilation therapy device monitoring device parameters, and data obtained by the ventilation therapy device monitoring non-device parameters. For example, for an air purifier, the monitoring data may be data, obtained by the air purifier, on air quality in a surrounding environment. For example, for a ventilator, the monitoring data may be data obtained by the ventilation therapy device monitoring conditions of the user. This is by way of example only, and some embodiments of the present application are not limited thereto.

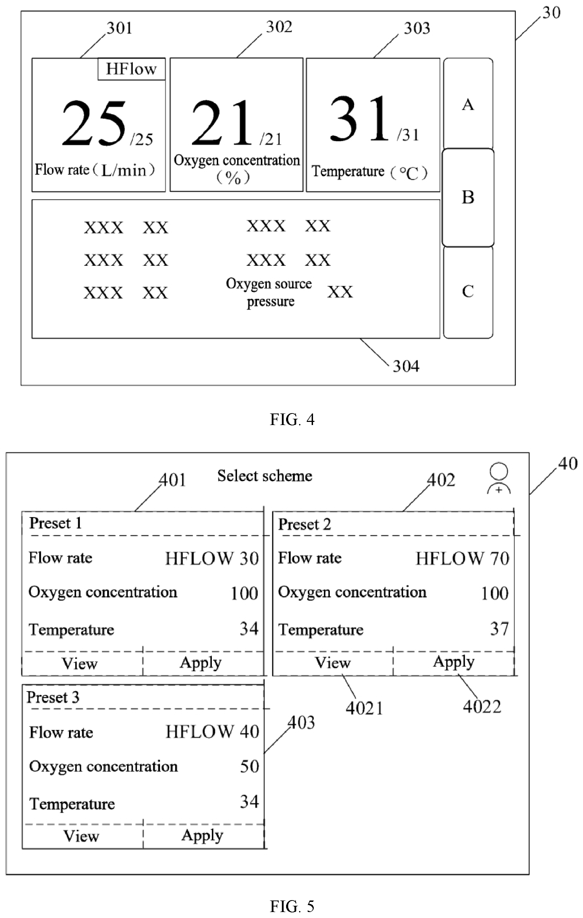

FIG. 4 is a schematic diagram showing a second display interface according to some embodiments. As shown in FIG. 4, target parameter controls in the second display interface 30 include: a flow rate parameter control 301, an oxygen concentration parameter control 302, and a temperature parameter control 303. The target parameter controls display a parameter setting value and a latest monitoring value of the corresponding target parameters, in the form of "latest monitoring value/parameter setting value", of which the latest monitoring value may be displayed in an enlarged manner. Specifically, the flow rate parameter control 301 includes a flow rate setting value and a flow rate monitoring value, the oxygen concentration parameter control 302 includes an oxygen concentration setting value and an oxygen concentration monitoring value, and the temperature parameter control 303 includes a temperature setting value and a temperature monitoring value. In addition, the target parameter controls further include a unit corresponding to the target parameters, such as a unit of flow rate, a unit of oxygen concentration, and a unit of temperature. Referring to FIG. 4, the second display interface 30 further includes a scheme control A, a scheme control B, and a scheme control C, of which the scheme control B is highlighted, indicating that the parameter setting values displayed by each target parameter control in the second display interface 30 are determined according to parameter setting values of target parameters in a parameter scheme B corresponding to the scheme control B. In some embodiments, in the case where the scheme control B is associated with the HFlow mode of the ventilation therapy device, in response to the first trigger operation by the user on the scheme control B, the ventilation therapy device is adjusted to operate in the HFlow mode, and the second display interface 30 is displayed. A mode label "HFlow" of the HFlow mode may be displayed in the flow rate parameter control 301 in the second display interface 30, indicating that the ventilation therapy device is currently in the HFlow mode. In addition, the data display area of the second display interface 30 further includes a monitoring data control 304. The monitoring data control 304 displays monitoring data, e.g., oxygen source pressure, of the ventilation therapy device.

In some embodiments of the present application, after the parameter adjustment is performed in response to the first trigger operation for the ventilation therapy device according to the target parameter scheme corresponding to the target scheme control, the second display interface is displayed. The second display interface includes the target parameter control corresponding to the target parameter, and the target parameter control is configured to display the parameter setting value and the latest monitoring value of the corresponding target parameter. In this way, the parameter setting value and the latest monitoring value of the target parameter displayed by the target parameter control can intuitively show an adjustment result of the target parameter of the ventilation therapy device, allowing the user to conveniently view the latest monitoring value of the target parameter of the current ventilation therapy device in the second display interface in a visualized manner. Thus, a human-machine interaction effect between the user and the ventilation therapy device can be improved.

In some embodiments, the first display interface further includes a create control, and the method further includes the following steps.

Step 204: Receive a second trigger operation by the user on the create control.

Step 205: Display a scheme selection interface in response to the second trigger operation, where the scheme selection interface includes a preset scheme control, and the preset scheme control includes a parameter settings list.

Step 206: Receive a third trigger operation by the user on the preset scheme control.

Step 207: Perform, in response to the third trigger operation, parameter adjustment for the ventilation therapy device according to the parameter settings list of the preset scheme control.

In some embodiments of the present application, the create control may be configured to create a new user of the ventilation therapy device. For a specific form of the second trigger operation, reference may be made to the related description of the first trigger operation in step 101. This is not limited in some embodiments of the present application. The preset scheme control is configured to display default values of the target parameters preset in the parameter settings list. The number of preset scheme controls displayed in the scheme selection interface may be one or more, and is not limited in some embodiments of the present application. The user may select and trigger a preset scheme control in the scheme selection interface to select a corresponding parameter scheme for the new user, and start device operation.

In some embodiments, the preset scheme control may include an apply sub-control, and step 206 may include the following step: receiving the third trigger operation by the user on the apply sub-control.

In some embodiments of the present application, for a specific form of the third trigger operation, reference may be made to the related description of the first trigger operation in step 101. This is not limited in some embodiments of the present application. In response to the third trigger operation by the user on the apply sub-control, parameter adjustment may be performed for the ventilation therapy device according to the parameter settings list of the preset scheme control to which the apply sub-control belongs. Specifically, the parameter adjustment may be performed for the ventilation therapy device according to default values of the target parameters in the parameter settings list of the preset scheme control.

FIG. 5 is a schematic diagram showing a scheme selection interface according to some embodiments. As shown in FIG. 5, the scheme selection interface 40 includes three preset scheme controls: a preset scheme control 401, i.e., Preset 1, a preset scheme control 402, i.e., Preset 2, and a preset scheme control 403, i.e., Preset 3. The parameter settings list includes flow rate, oxygen concentration, and temperature parameters, and default values corresponding to the parameters. For example, in Preset 1, the parameter settings list includes: a flow rate of 30 L/min in the HFlow mode, an oxygen concentration of 100%, and a temperature of 34°C. The preset scheme control may further include an apply sub-control and a view sub-control. Upon receiving a trigger operation by the user on the view sub-control, the parameter settings list may be expanded for display. Upon receiving third trigger operation by the user on the apply sub-control, parameter adjustment may be performed for the ventilation therapy device according to default values of the target parameters in the parameter settings list of the preset scheme control. For example, the preset scheme control 402 may include a view sub-control 4021 and an apply sub-control 4022.

In some embodiments of the present application, the second trigger operation by the user on the create control of the first display interface is received, and in response to the second trigger operation, the scheme selection interface is displayed. Because the scheme selection interface includes the preset scheme control, and the preset scheme control includes the parameter settings list, the user can more intuitively view the preset parameter scheme in the parameter settings list of the preset scheme control. The third trigger operation by the user on the preset scheme control is received, and in response to the third trigger operation, the parameter adjustment is performed for the ventilation therapy device according to the parameter settings list of the preset scheme control. In this way, the user can conveniently select one of the preset parameter settings lists as a newly created parameter scheme to perform parameter adjustment for the ventilation therapy device, thereby enhancing practicality of the parameter adjustment method according to some embodiments of the present application.

In some embodiments, the first display interface further includes an alarm control, and the method further includes the following steps.

Step 208: Receive a fourth trigger operation by the user on the alarm control.

Step 209: Display an alarm settings interface in response to the fourth trigger operation, where the alarm settings interface includes an alarm item control.

Step 210: Receive a fifth trigger operation by the user on the alarm item control.

Step 211: Update a parameter alarm threshold corresponding to the alarm item control in response to the fifth trigger operation.

In some embodiments of the present application, the alarm control may be configured to perform alarm setting for the ventilation therapy device. For a specific form of the fourth trigger operation and the fifth trigger operation, reference may be made to the related description of the first trigger operation in step 101. This is not limited in some embodiments of the present application. The alarm item control is configured to display the target parameter and an alarm threshold corresponding to the target parameter. The number of alarm item controls displayed in the alarm settings interface may be one or more, and the user may select, according to needs, a target parameter that requires real-time data monitoring and threshold-exceeding alarms. This is not limited in some embodiments of the present application.

In some embodiments of the present application, the alarm item control may include a modify sub-control and a confirm sub-control. A trigger operation by the user on the modify sub-control may be received to modify the parameter alarm threshold, and then a trigger operation by the user on the confirm sub-control may be received to update the parameter alarm threshold corresponding to the alarm item control. The user may perform the trigger operation on the modify sub-control using an input device connected to the ventilation therapy device. For example, the input device connected to the ventilation therapy device may be a mouse or a keyboard. The user may manipulate the mouse to click the modify sub-control, invoking a virtual keypad and displaying it on the alarm settings interface. The user may modify the parameter alarm threshold by clicking with the mouse or tapping the touch screen. Alternatively, the user may trigger the modify sub-control by entering a shortcut via the keyboard, and enter a new parameter alarm threshold via the keyboard to modify the parameter alarm threshold. This is by way of example only, and some embodiments of the present application are not limited thereto.

In a feasible implementation, the first display interface may include an alarm information area. When real-time data monitored by the ventilation therapy device indicates that a latest monitoring value of a target parameter exceeds the parameter alarm threshold, the parameter setting value and the latest monitoring value of the target parameter may be displayed in the alarm information area. The parameter alarm threshold may include an upper threshold and a lower threshold. The upper threshold indicates that the latest monitoring value of the target parameter should not exceed the threshold, and the lower threshold indicates that the latest monitoring value of the target parameter should not fall below the threshold.

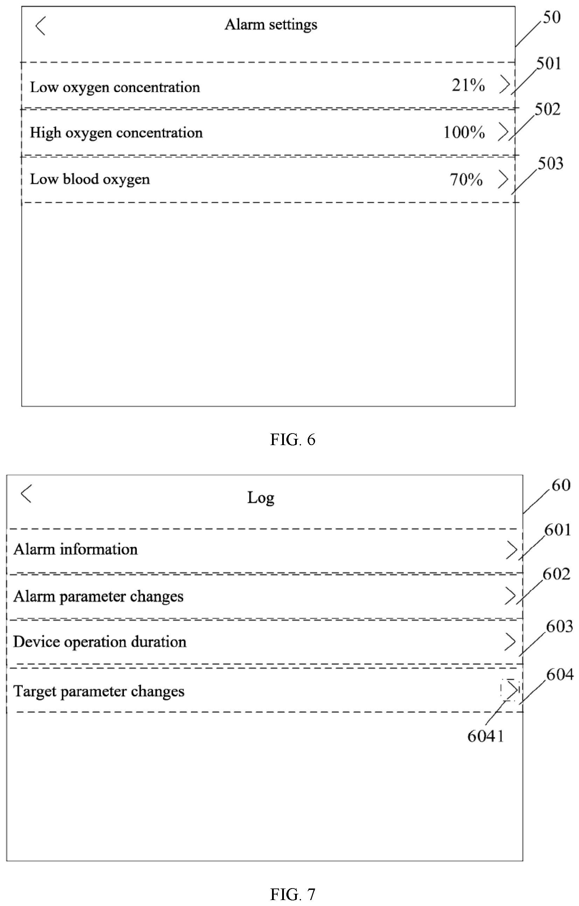

FIG. 6 is a schematic diagram showing an alarm settings interface according to some embodiments. As shown in FIG. 6, the alarm settings interface 50 includes alarm item controls: a low oxygen concentration control 501, a high oxygen concentration control 502, and a low blood oxygen control 503. When real-time data of oxygen concentration or blood oxygen monitored by the ventilation therapy device indicates that the latest monitoring value of the oxygen concentration or the blood oxygen exceeds the parameter alarm threshold, alarm information may be displayed in the alarm information area of the first display interface to prompt the user to eliminate the risk, thereby improving safety of the parameter adjustment method according to some embodiments of the present application.

In some embodiments of the present application, the fourth trigger operation by the user on the alarm control of the first display interface is received, and in response to the fourth trigger operation, the alarm settings interface is displayed. Because the alarm settings interface includes the alarm item control, the user can conveniently view the current alarm items of the ventilation therapy device. The fifth trigger operation by the user on the alarm item control is received, and in response to the fifth trigger operation, the parameter alarm threshold corresponding to the alarm item control is updated, so that the user can conveniently update the parameter alarm threshold of the alarm item. Thus, safety of the parameter adjustment method according to some embodiments of the present application can be improved.

In some embodiments, the first display interface further includes a log control, and the method further includes the following steps.

Step 212: Receive a sixth trigger operation by the user on the log control.

Step 213: Display a log interface in response to the sixth trigger operation, where the log interface includes a log item control.

Step 214: Receive a seventh trigger operation by the user on the log item control.

Step 215: Display log item information corresponding to the log item control in response to the seventh trigger operation.

In some embodiments of the present application, for a specific form of the sixth trigger operation and the seventh trigger operation, reference may be made to the related description of the first trigger operation in step 101. This is not limited in some embodiments of the present application. The log item control is configured to display an item name of a log item, for example, alarm information, alarm parameter changes, device operation duration, target parameter changes, power on/off information statistics, device self-check information, device user changes, etc.

In some embodiments of the present application, in response to the seventh trigger operation by the user on the log item control, an item information interface may be displayed, and log item information corresponding to the log item control may be displayed in the item information interface. The item information interface may replace the log interface and is displayed on the display screen of the ventilation therapy device. Alternatively, the item information interface may be displayed in the log interface in a floating manner. This is by way of example only, and some embodiments of the present application are not limited thereto.

FIG. 7 is a schematic diagram showing a log interface according to some embodiments. As shown in FIG. 7, the log interface 60 includes log item controls: alarm information 601, alarm parameter changes 602, device operation duration 603, and target parameter changes 604. The log item controls may include an expansion display control. The user may call out an item information interface through a trigger operation on the expansion display control, to view log item information displayed in the item information interface. For example, the log item control of target parameter changes 604 may include an expansion display control 6041.

In some embodiments of the present application, the sixth trigger operation by the user on the log control is received, and in response to the sixth trigger operation, the log interface is displayed, where the log interface includes the log item control. The seventh trigger operation by the user on the log item control is received, and in response to the seventh trigger operation, the log item information corresponding to the log item control is displayed. In this way, the user can conveniently view log items of the ventilation therapy device, so that the user can intuitively view specific information of the log items of the ventilation therapy device, thereby improving a human-machine interaction effect between the user and the ventilation therapy device.

In some embodiments, the first display interface further includes a trend chart control, and the method further includes the following steps.

Step 216: Receive an eighth trigger operation by the user on the trend chart control.

Step 217: Display a trend chart interface in response to the eighth trigger operation, where the trend chart interface includes: a time interval control and a parameter graph control corresponding to the target parameter.

Step 218: Receive a ninth trigger operation by the user on the time interval control.

Step 219: Display, in the parameter graph control in response to the ninth trigger operation, a parameter value variation trend of the corresponding target parameter over a target time interval, where the target time interval is a time interval currently displayed by the time interval control.

In some embodiments of the present application, the user may call out the trend chart interface through the eighth trigger operation on the trend chart control, and view, in the trend chart interface, a variation trend chart of the target parameter and/or monitoring data of the ventilation therapy device. For a specific form of the eighth trigger operation and the ninth trigger operation, reference may be made to the related description of the first trigger operation in step 101. This is not limited in some embodiments of the present application. The time interval control is configured to limit a time interval of data participating in parameter value variation trend analysis. The number of parameter graph controls corresponding to the target parameter displayed in the trend chart interface may be one or more. The user may select, according to needs, a target parameter that requires variation trend analysis and graphical representation, and associate the target parameter with the corresponding parameter graph control. This is not limited in some embodiments of the present application.

In some embodiments of the present application, when the user selects a target parameter, the ninth trigger operation by the user on the time interval control may be received. In response to the ninth trigger operation, the time interval currently displayed in the time interval control is determined as the target time interval, and in the parameter graph control corresponding to the target parameter, a parameter value variation trend of the target parameter over the target time interval is displayed. The parameter graph control may include a horizontal axis and a vertical axis. The horizontal axis may represent time, and a coordinate interval may be determined according to the target time interval. The vertical axis may represent a parameter value of the target parameter. A graphical representation form of the parameter graph control may be a bar chart or a line chart, and is not limited in some embodiments of the present application.

In another feasible implementation, when the user selects a target time interval, a tenth trigger operation by the user on the parameter graph control corresponding to the target parameter may be received. In response to the tenth trigger operation, in the parameter graph control corresponding to the target parameter, a parameter value variation trend of the target parameter over the target time interval is displayed. A specific implementation may be selected according to needs of the user, and is not limited in some embodiments of the present application.

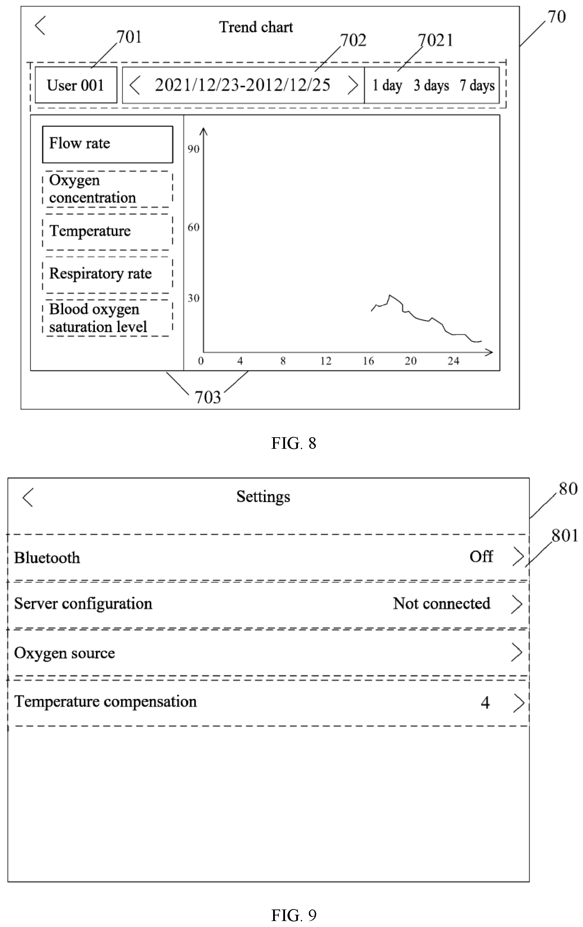

FIG. 8 is a schematic diagram showing a trend chart interface according to some embodiments. As shown in FIG. 8, the trend chart interface 70 includes a user identity control 701, a time interval control 702, and respective parameter graph controls 703 for a plurality of target parameters. The user identity control 701 displays a user identity of "User 001", indicating that data of the current trend chart belongs to User 001. The time interval control 702 displays a time interval of year, month, and day. The time interval control 702 further includes a shortcut sub-control 7021. By receiving a trigger operation by the user on the shortcut sub-control 7021, an interval difference of the time interval displayed by the time interval control 702 may be fixed to a specified duration, such as 1 day, 3 days, or 7 days as shown in FIG. 8.

In some embodiments of the present application, the eighth trigger operation by the user on the trend chart control of the first display interface is received, and in response to the eighth trigger operation, the trend chart interface is displayed. The trend chart interface includes: the time interval control and the parameter graph control corresponding to the target parameter. The ninth trigger operation by the user on the time interval control is received. In response to the ninth trigger operation, the parameter value variation trend of the corresponding target parameter over the target time interval is displayed in the parameter graph control, where the target time interval is the time interval currently displayed by the time interval control. In this way, the user can conveniently select, in the trend chart interface, a target parameter for which a variation trend needs to be viewed, and select a time interval of data to be analyzed, thereby viewing, through the parameter graph control of the target parameter, the parameter value variation trend of the target parameter over the target time interval in a visualized manner. Thus, a visual display effect of the ventilation therapy device can be improved, and experience of the user using the device can be enhanced.

In some embodiments, the first display interface further includes a settings control, and the method further includes:
receiving a settings trigger operation by the user on the settings control;
displaying a settings interface in response to the settings trigger operation, where the settings interface includes a setting item control;
receiving a view trigger operation by the user on the setting item control; and
displaying setting item information corresponding to the setting item control in response to the view trigger operation.

FIG. 9 is a schematic diagram showing a settings interface according to some embodiments. As shown in FIG. 9, the settings interface 80 includes a plurality of setting items of the ventilation therapy device, such as Bluetooth switch, server configuration, oxygen source, temperature compensation, etc. The user may view information about the setting items in the settings interface 80, and may further modify the setting information by triggering a setting item control 801.

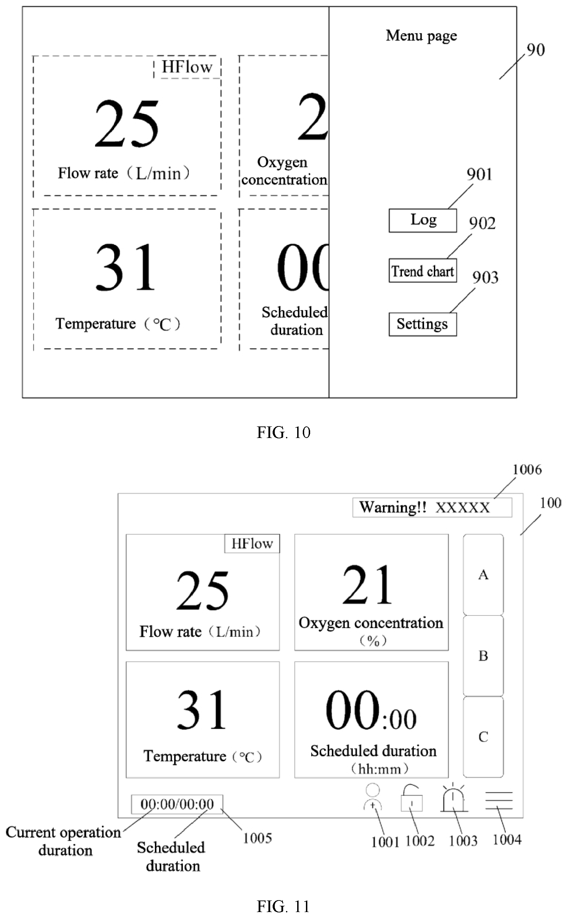

In some embodiments, the first display interface may include a menu control. The log control, the trend chart control, the settings control, etc. may be integrated into a menu page corresponding to the menu control, and only the menu control is displayed on the first display interface. This can save space on the first display interface, making the interface more tidy and aesthetically pleasing, thereby enhancing the visual effect of the first display interface. A trigger operation by the user on the menu control of the first display interface may be received to display the menu page. Referring to FIG. 10, the menu page 90 includes a log control 901, a trend chart control 902, and a settings control 903. The menu page 90 may be displayed in the first display interface in a floating manner. Alternatively, the first display interface may be resized so that the display screen of the ventilation therapy device displays the first display interface and the menu page simultaneously. This is by way of example only, and some embodiments of the present application are not limited thereto.

FIG. 11 is a second schematic diagram showing a first display interface according to some embodiments. As shown in FIG. 11, compared with FIG. 2, the first display interface 100, in a lower-right corner thereof, further includes: a create control 1001, a lock control 1002, an alarm control 1003, and a menu control 1004. A trigger operation by the user on the lock control 1002 may be received to lock the first display interface 100. After the first display interface is locked, the ventilation therapy device cannot receive a trigger operation on each of the controls in the first display interface 100. In this way, an inadvertent touch on the first display interface 100 by an unauthorized user can be prevented, thereby improving safety of the parameter adjustment method according to some embodiments of the present application. Referring to FIG. 11, the lower-left corner of the first display interface 100 further includes a duration display area 1005, displaying a current operation duration and a preset duration of the ventilation therapy device. The upper-right corner of the first display interface further includes an alarm information area 1006 configured to display alarm information.

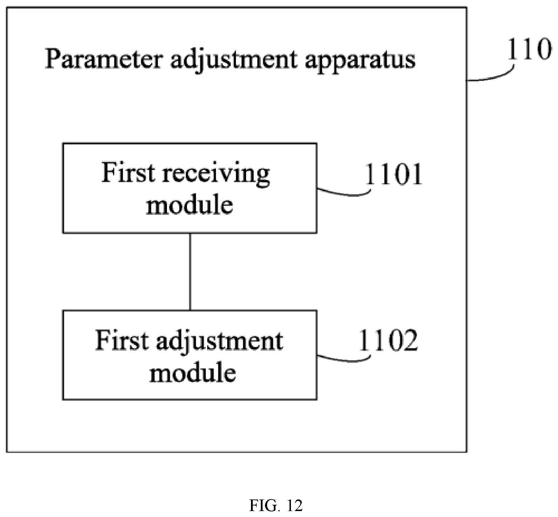

FIG. 12 is a block diagram showing a parameter adjustment apparatus according to some embodiments. As shown in FIG. 12, the parameter adjustment apparatus is applied to a ventilation therapy device. The parameter adjustment apparatus 110 includes:

A first receiving module 1101 configured to receive a first trigger operation by a user on a target scheme control, where a first display interface of the ventilation therapy device includes at least one scheme control, and the target scheme control is one of the at least one scheme control.

A first adjustment module 1102 configured to perform, in response to the first trigger operation, parameter adjustment for the ventilation therapy device according to a target parameter scheme corresponding to the target scheme control, where the target parameter scheme includes a target parameter and a parameter setting value corresponding to the target parameter.

After performing, in response to the first trigger operation, the parameter adjustment for the ventilation therapy device according to the target parameter scheme corresponding to the target scheme control, the method further includes:
displaying a second display interface in response to the first trigger operation, where the second display interface includes a target parameter control corresponding to the target parameter, and the target parameter control is configured to display a parameter setting value and a latest monitoring value of the corresponding target parameter.

In some embodiments, the ventilation therapy device includes at least one parameter mode; the ventilation therapy device, in any one of the parameter modes, automatically operates according to a corresponding preset parameter scheme; and the first adjustment module 1102 is specifically configured to:
adjust the ventilation therapy device to operate in a target parameter mode in response to the first trigger operation, where the target parameter mode is a parameter mode, among the at least one parameter mode, that is associated with the target scheme control.

In some embodiments, the first display interface further includes a create control, and the apparatus 110 further includes:
a second receiving module configured to receive a second trigger operation by the user on the create control;
a first display module configured to display a scheme selection interface in response to the second trigger operation, where the scheme selection interface includes a preset scheme control, and the preset scheme control includes a parameter settings list;
a third receiving module configured to receive a third trigger operation by the user on the preset scheme control; and
a second adjustment module configured to perform, in response to the third trigger operation, parameter adjustment for the ventilation therapy device according to the parameter settings list of the preset scheme control.

In some embodiments, the first display interface further includes an alarm control, and the apparatus 110 further includes:
a fourth receiving module configured to receive a fourth trigger operation by the user on the alarm control;
a second display module configured to display an alarm settings interface in response to the fourth trigger operation, where the alarm settings interface includes an alarm item control;
a fifth receiving module configured to receive a fifth trigger operation by the user on the alarm item control; and
an update module configured to update a parameter alarm threshold corresponding to the alarm item control in response to the fifth trigger operation.

In some embodiments, the first display interface further includes a log control, and the apparatus 110 further includes:
a sixth receiving module configured to receive a sixth trigger operation by the user on the log control;
a third display module configured to display a log interface in response to the sixth trigger operation, where the log interface includes a log item control;
a seventh receiving module configured to receive a seventh trigger operation by the user on the log item control; and
a fourth display module configured to display log item information corresponding to the log item control in response to the seventh trigger operation.

In some embodiments, the first display interface further includes a trend chart control, and the apparatus 110 further includes:
an eighth receiving module configured to receive an eighth trigger operation by the user on the trend chart control;
a fifth display module configured to display a trend chart interface in response to the eighth trigger operation, where the trend chart interface includes: a time interval control and a parameter graph control corresponding to the target parameter;
a ninth receiving module configured to receive a ninth trigger operation by the user on the time interval control; and
a sixth display module configured to display, in the parameter graph control in response to the ninth trigger operation, a parameter value variation trend of the corresponding target parameter over a target time interval, where the target time interval is a time interval currently displayed by the time interval control.

The apparatus embodiment is basically similar to the method embodiment, and therefore is described simply. For a related part, reference may be made to the part of the description of the method embodiment.

The parameter adjustment apparatus has the same advantages over the prior art as the aforementioned parameter adjustment method, and details are not described herein again.

According to some embodiments of the present application, a ventilation therapy device is provided. The ventilation therapy device is equipped with the parameter adjustment apparatus as described above in some embodiments, and is configured to perform the parameter adjustment method as described above in some embodiments.

According to some embodiments of the present application, a storage medium is further provided. Instructions in the storage medium, when executed by a processor of an electronic device, cause the electronic device to perform the steps in the parameter adjustment method according to some of the above-described embodiments.

User information (including but not limited to user device information, user personal information, etc.) and related data involved in the present application are information authorized by the user or authorized by various parties.

A person skilled in the art may easily figure out another implementation of the present application after considering the description and practicing the invention disclosed herein. The present application is intended to cover any variations, uses, or adaptive changes of the present application. These variations, uses, or adaptive changes follow the general principles of the present application and include common knowledge or common technical means in the art which are not disclosed in the present application. The description and some embodiments are merely considered as examples, and the actual scope and spirit of the present application are pointed out in the following claims.

It should be understood that the present application is not limited to the precise structures described above and shown in the accompanying drawings, and various modifications and changes may be made without departing from the scope thereof. The scope of the present application is merely defined by the appended claims.

## Claims

1. A parameter adjustment method, **characterized in that** the method is applied to a ventilation therapy device and comprises:
receiving a first trigger operation by a user on a target scheme control, wherein a first display interface of the ventilation therapy device comprises at least one scheme control, and the target scheme control is one of the at least one scheme control; and
performing, in response to the first trigger operation, parameter adjustment for the ventilation therapy device according to a target parameter scheme corresponding to the target scheme control, wherein the target parameter scheme comprises a target parameter and a parameter setting value corresponding to the target parameter.

2. The method according to claim 1, **characterized in that** after performing, in response to the first trigger operation, the parameter adjustment for the ventilation therapy device according to the target parameter scheme corresponding to the target scheme control, the method further comprises:
displaying a second display interface in response to the first trigger operation, wherein the second display interface comprises a target parameter control corresponding to the target parameter, and the target parameter control is configured to display the parameter setting value and a latest monitoring value of the corresponding target parameter.

3. The method according to claim 1, **characterized in that** the ventilation therapy device comprises at least one parameter mode; the ventilation therapy device, in any one of the parameter modes, automatically operates according to a corresponding preset parameter scheme; and performing, in response to the first trigger operation, the parameter adjustment for the ventilation therapy device according to the target parameter scheme corresponding to the target scheme control comprises:
adjusting the ventilation therapy device to operate in a target parameter mode in response to the first trigger operation, wherein the target parameter mode is a parameter mode, among the at least one parameter mode, that is associated with the target scheme control.

4. The method according to claim 1, **characterized in that** the first display interface further comprises a create control, and the method further comprises:
receiving a second trigger operation by the user on the create control;
displaying a scheme selection interface in response to the second trigger operation, wherein the scheme selection interface comprises a preset scheme control, and the preset scheme control comprises a parameter settings list;
receiving a third trigger operation by the user on the preset scheme control; and
performing, in response to the third trigger operation, parameter adjustment for the ventilation therapy device according to the parameter settings list of the preset scheme control.

5. The method according to claim 1, **characterized in that** the first display interface further comprises an alarm control, and the method further comprises:
receiving a fourth trigger operation by the user on the alarm control;
displaying an alarm settings interface in response to the fourth trigger operation, wherein the alarm settings interface comprises an alarm item control;
receiving a fifth trigger operation by the user on the alarm item control; and
updating a parameter alarm threshold corresponding to the alarm item control in response to the fifth trigger operation.

6. The method according to claim 1, **characterized in that** the first display interface further comprises a log control, and the method further comprises:
receiving a sixth trigger operation by the user on the log control;
displaying a log interface in response to the sixth trigger operation, wherein the log interface comprises a log item control;
receiving a seventh trigger operation by the user on the log item control; and
displaying log item information corresponding to the log item control in response to the seventh trigger operation.

7. The method according to claim 1, **characterized in that** the first display interface further comprises a trend chart control, and the method further comprises:
receiving an eighth trigger operation by the user on the trend chart control;
displaying a trend chart interface in response to the eighth trigger operation, wherein the trend chart interface comprises: a time interval control and a parameter graph control corresponding to the target parameter;
receiving a ninth trigger operation by the user on the time interval control; and
displaying, in the parameter graph control in response to the ninth trigger operation, a parameter value variation trend of the corresponding target parameter over a target time interval, wherein the target time interval is a time interval currently displayed by the time interval control.

8. A parameter adjustment apparatus, **characterized in that** the apparatus is applied to a ventilation therapy device and comprises:
a first receiving module configured to receive a first trigger operation by a user on a target scheme control, wherein a first display interface of the ventilation therapy device comprises at least one scheme control, and the target scheme control is one of the at least one scheme control; and
a first adjustment module configured to perform, in response to the first trigger operation, parameter adjustment for the ventilation therapy device according to a target parameter scheme corresponding to the target scheme control, wherein the target parameter scheme comprises a target parameter and a parameter setting value corresponding to the target parameter.

9. A ventilation therapy device, **characterized in that** the ventilation therapy device is equipped with the parameter adjustment apparatus according to claim 8, and is configured to perform the parameter adjustment method according to any one of claims 1 to 7.

10. A storage medium, **characterized in that** instructions in the storage medium, when executed by a processor of an electronic device, cause the electronic device to perform the parameter adjustment method according to any one of claims 1 to 7.
